(19) Europäisches Patentamt / European Patent Office / Office européen des brevets

(11) **EP 4 176 919 A1**

(12) **EUROPEAN PATENT APPLICATION**
published in accordance with Art. 153(4) EPC

(43) Date of publication:
**10.05.2023 Bulletin 2023/19**

(21) Application number: **21834083.4**

(22) Date of filing: **02.07.2021**

(51) International Patent Classification (IPC):
**A61M 25/00** (2006.01)   **F16L 11/04** (2006.01)
**F16L 11/24** (2006.01)

(52) Cooperative Patent Classification (CPC):
**A61M 25/00; F16L 11/04; F16L 11/24**

(86) International application number:
**PCT/JP2021/025225**

(87) International publication number:
**WO 2022/004893 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.07.2020   JP 2020115022**

(71) Applicant: **Junkosha Inc.**
**Kasama-shi, Ibaraki 309-1603 (JP)**

(72) Inventors:
• **TANABE, Suguru**
**Kasama-shi Ibaraki 309-1603 (JP)**
• **YAMADA, Naoya**
**Kasama-shi Ibaraki 309-1603 (JP)**

(74) Representative: **Daub, Thomas**
**Patent- und Rechtsanwaltskanzlei Daub**
**Bahnhofstrasse 5**
**88662 Überlingen (DE)**

(54) **TUBE**

(57) The invention relates to a tube including at least one layer formed of polytetrafluoroethylene, and an object of the invention is to provide a tube in which flexibility is adjusted while high mechanical strength is maintained. The object can be appropriately achieved by a tube including at least one layer formed by spirally winding a PTFE film, in which the tube has an endothermic peak in a range of 380°C ± 10°C in a temperature increasing process of DSC of the tube, and in at least one of the at least one layer, the number of windings per length of 10 mm in a longitudinal direction is not constant.

FIG. 2

EP 4 176 919 A1

**Description**

TECHNICAL FIELD

The present invention relates to a tube using polytetrafluoroethylene (hereinafter, referred to as "PTFE").

BACKGROUND ART

There is a product provided with a fluorine resin lining on an inner layer of a tubular body, such as a fluid transport tube that is required to be used for medical applications and requires chemical resistance. In particular, when the tubular body is used for applications requiring chemical resistance, cleanability, non-adhesiveness, low friction, and the like, PTFE is suitable as a material for the lining. For example, it is known that in order to improve slidability of an inner surface of a catheter, a PTFE liner tube is disposed in an innermost layer of the catheter.

A catheter used for an intravascular surgery or the like needs to be percutaneously inserted into a body to pass through a blood vessel and cause a catheter distal end to reach a lesion site, and thus straightness for moving straight in the blood vessel, operation transmissibility for transmitting an operation of an operator, flexibility for reducing a burden on a patient, and the like are required. In order to satisfy all the requirements, the catheter is implemented by laminating layers having different characteristics, and among the layers, an innermost PTFE liner tube is required to be excellent in mechanical strength such as high tensile strength, extensibility, crack resistance, pressure resistance, and buckling resistance while being thin.

As one of methods for producing a PTFE liner tube, there is a method in which a PTFE dispersion is dip-coated on a core wire such as a metal wire, dried and sintered, and then the core wire is pulled out and molded (see, for example, Patent Literature 1). There is a method in which a paste obtained by mixing a PTFE powder and an organic solvent called an auxiliary agent is extruded on a core wire, dried and sintered, and then the core wire is pulled out and molded (for example, see Patent Literature 2). The PTFE liner tube molded by the method according to Patent Literature 1 or Patent Literature 2 is low in mechanical strength, and when being used for an innermost layer of a catheter, there is a possibility that troubles such as damages such as tearing or breakages of a PTFE liner, extension of the liner tube, reduction in an inner diameter, and biting of an inserted object may occur due to friction between an inner surface of the catheter and the inserted object (a treatment tool or the like).

As another method for producing a PTFE liner tube, there is a method in which a paste obtained by mixing a PTFE powder and an auxiliary agent is extruded into a tube shape, and then the molded liner tube is extended in a longitudinal direction to make the liner tube thinner to improve tensile strength of the tube (see Patent Literature 3). The PTFE liner tube molded by this method has a problem in that the PTFE liner tube is high in tensile strength, but is poor in extensibility and poor in flexibility. The tube poor in flexibility requires a strong operating force when a bending radius is reduced, and is easily buckled due to hardness, which causes troubles such as blocking of the tube and biting of an inserted object due to buckling. Patent Literature 4 discloses a thin PTFE tube with high tensile strength and extensibility, but for applications requiring higher performance, the tube is insufficient in flexibility.

Further, there is a catheter having a tapered shape in order to improve bendability of a distal end portion of the catheter. A PTFE liner tube used for the catheter having a tapered shape is used by covering a core wire having a tapered shape with the tube being extended and reduced in diameter. By forming the tube into a tapered shape, the bendability of the tapered shape portion is improved, but flexibility is reduced due to extension of the PTFE tube, and an expected effect cannot be sufficiently obtained.

PRIOR ART LITERATURES

PATENT LITERATURES

Patent Literature 1: JP2000-316977A
Patent Literature 2: JP2013-176583A
Patent Literature 3: JP2004-340364A
Patent Literature 4: Japanese Patent No. 6244490

SUMMARY OF INVENTION

OBJECT TO BE ATTAINED BY THE INVENTION

In view of the above-mentioned problem, an object of the invention is to provide a thin tube using PTFE, in which flexibility is adjusted while high tensile strength is maintained.

MEANS FOR ATTAINING THE OBJECT

The object can be achieved by a tube including at least one layer formed by spirally winding a PTFE film, in which the tube has an endothermic peak in a range of 380°C $\pm$ 10°C in a temperature increasing process of DSC of the tube, and in at least one of the at least one layer, the number of windings per length of 10 mm in a longitudinal direction is not constant.
In a case in which the at least one layer formed by spirally winding the PTFE film includes two or more layers, it is preferable that at least one layer is spirally wound clockwise, and at least one layer is spirally wound counterclockwise.
It is preferable that the at least one layer has an average thickness of 3 $\mu$m or more and 75 $\mu$m or less.
It is preferable that tensile strength of the tube obtained by a tensile test according to JIS K7127-1999 is 100 N/mm$^2$ or more.
The object of the invention can be achieved by a tube including at least one layer formed by spirally winding a PTFE film, in which the tube has an endothermic peak in a range of 380°C $\pm$ 10°C in a temperature increasing process of DSC of the tube, and in at least one of the at least one layer, a maximum number of windings per length of 10 mm in a longitudinal direction is different from a minimum number of windings per length of 10 mm in the longitudinal direction by at least 0.1 windings/10 mm or more.

EFFECTS OF INVENTION

With the above-recited configuration, flexibility in the longitudinal direction of the tube can be adjusted while high tensile strength is maintained in the entire tube, whereby a tube excellent in pliability can be obtained. The tube according to the invention can be implemented to be thin, and is suitable as a liner tube of a tubular body which requires flexibility and smaller diameter.

BRIEF DESCRIPTION OF DRAWINGS

Fig. 1 is a diagram illustrating a conventional tube.
Fig. 2 illustrates diagrams illustrating examples of a tube according to the invention.
Fig. 3 illustrates schematic views illustrating structures of a PTFE layer of the tube according to the invention.
Fig. 4 is a diagram illustrating a winding structure of the PTFE film of the tube according to the invention.
Fig. 5 is a diagram illustrating parameters related to the tube according to the invention.
Fig. 6 is a diagram illustrating a method for counting the number of windings of the film of the tube according to the invention.
Fig. 7 illustrates diagrams illustrating the number of windings of the film of the tube according to the invention.

EMBODIMENTS FOR CARRYING OUT THE INVENTION

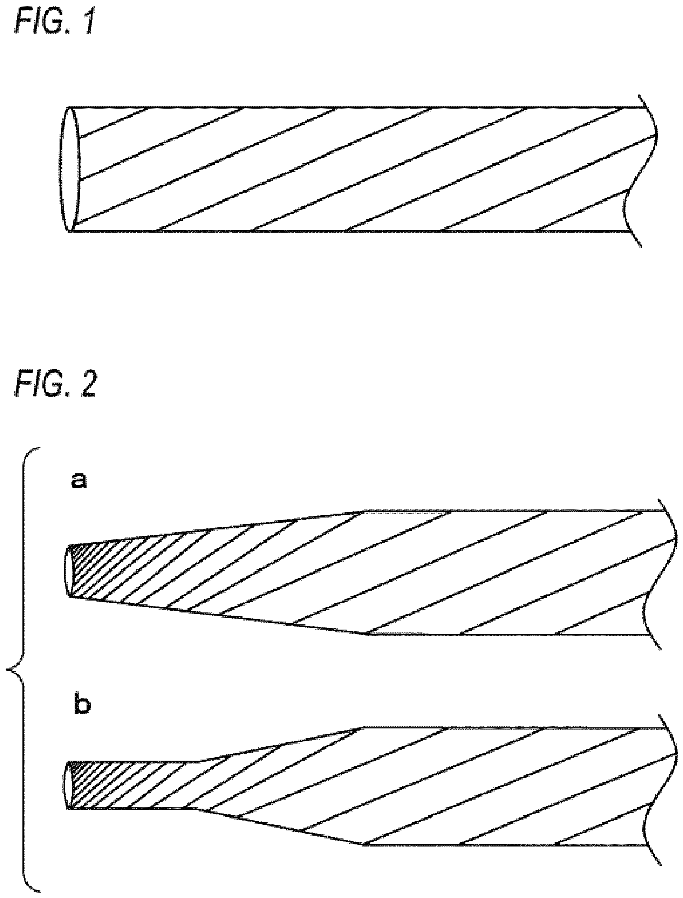

Fig. 1 is a diagram illustrating a conventional tube produced by spirally winding a film. The conventional tube is formed by winding the film for a constant number of windings over an entire length of the tube. A tube according to the invention preferably includes a portion in which the number of windings of a film is not constant in a longitudinal direction. Hereinafter, a tube according to a preferred embodiment of the invention will be described.
Fig. 2 illustrates diagrams illustrating examples of the tube according to the invention. Tubes illustrated in a and b of Fig. 2 are tubes including at least one layer formed by spirally winding a PTFE film. In the examples illustrated in Fig. 2, at least an outermost layer of the tube is formed by spirally winding the PTFE film, and the number of windings of the film on a left side is larger than the number of windings of the film on a right side. Accordingly, flexibility on the left side is improved while tensile strength of the entire tube is maintained. In addition to the layer, the tube according to the invention may further include a layer formed by spirally winding the PTFE film, a layer formed by a PTFE seamless film, a layer formed by cigarette winding the PTFE film, a layer formed by a resin other than PTFE, or the like. Fig. 2 illustrates examples of the tube having a shape in which an outer diameter on the left side of the tube is smaller than an outer diameter on the right side, but the invention is not limited thereto, and the outer diameter may be constant.

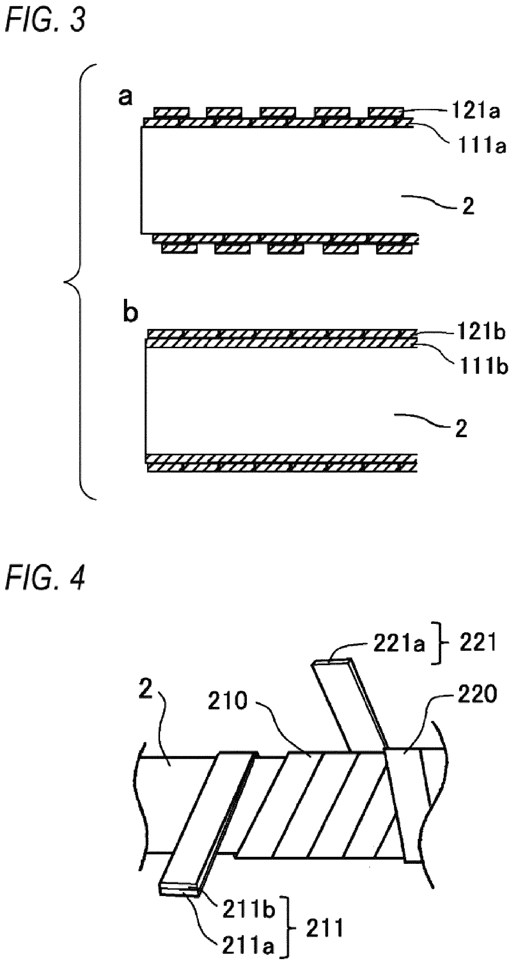

Regarding the tube including at least one layer formed by spirally winding the PTFE film according to the invention, Fig. 3 illustrates schematic views illustrating structures in which the PTFE film is wound. In the example illustrated in a of Fig. 3, a film 111a as a first layer is spirally wound on the outer periphery of a core wire 2, and a film 121a as a second layer is spirally wound outside the film 111a with a gap between windings. In the example illustrated in b of Fig. 3, a cylindrical PTFE film 111b as a first layer is disposed on the outer periphery of the core wire 2, and a film 121b as a second layer is spirally wound outside the PTFE film 111b. In the examples illustrated in Fig. 3, the at least one layer formed by spirally winding the PTFE film is configured by one layer or two layers, but in the tube according to the invention, the at least one layer may be configured by three or more layers. The tube according to the invention may be a tube in which a layer made of PTFE or a resin other than PTFE is laminated inside or outside of the at least one layer formed by spirally winding the PTFE film.

The at least one layer formed by spirally winding the PTFE film of the tube according to the invention preferably has a small average thickness. The average thickness of the entire layer formed by spirally winding the PTFE film of the tube according to the invention is preferably 100 $\mu$m or less, and an average thickness of one layer formed by spirally winding the PTFE film is preferably 3 $\mu$m or more and 75 $\mu$m or less. The average thickness of one layer is more preferably 5 $\mu$m or more and 50 $\mu$m or less, and still more preferably 5 $\mu$m or more and 40 $\mu$m or less.

Fig. 4 is a diagram illustrating a structure of the layer formed by spirally winding the PTFE film of the tube according to the invention. In the invention, the sentence "including one or more layers formed by spirally winding the PTFE film" means including one or more layers formed by spirally winding one PTFE film. In the invention, one film includes one formed by laminating a plurality of films and winding at the same angle. For example, in the example illustrated in Fig. 4, a first layer 210 is formed by spirally winding a PTFE film 211 on the outer periphery of the core wire 2. A second layer 220 is formed by spirally winding a PTFE film 221 outside the first layer 210. The PTFE film 211 is implemented by laminating one film 211a made of PTFE and one film 211b made of a thermoplastic fluororesin. The PTFE film 221 of the second layer 220 is implemented by one film 221a made of PTFE.

In the tube according to the invention, in a case in which the layer formed by spirally winding the film includes two or more layers, it is more preferable that at least one layer is wound clockwise, and at least one layer is wound counterclockwise. In the example illustrated in Fig. 4, the layer formed by spirally winding the film includes two layers. The film 211 of the first layer 210 is wound counterclockwise (S-winding), and the film 221 of the second layer 220 is wound clockwise (Z-winding). With such a structure, mechanical strength of the tube can be easily adjusted.

In the tube according to the invention, it is preferable that in at least one of the layer formed by spirally winding the PTFE film, the number of windings per length of 10 mm in the longitudinal direction is not constant. The number of windings per length of 10 mm in the longitudinal direction of the tube in one tube can be changed by, for example, changing a winding speed of the film while feeding the core wire at a constant speed in a step of winding the film around the core wire. At this time, when a core wire having a tapered shape is used, an adjustment can be easily made to change the number of windings of the film.

A film made of PTFE implementing the PTFE film used for the tube according to the invention is preferably made of high density PTFE. Containing high density PTFE is advantageous in achieving high airtightness and mechanical strength. The film made of high density PTFE can be produced, for example, as follows. A preform obtained by mixing a PTFE resin fine powder and an auxiliary agent (a lubricant such as solvent naphtha or white oil) and compressing is put into an extruder to be molded into a film shape, and a molded article is dried. When the film is dried, the auxiliary agent in the molded article molded into a film shape is volatilized, and an unsintered film made of PTFE having pores in the film is obtained. When the unsintered film made of PTFE is heated to a temperature equal to or higher than a melting point and sintered, the pores in the film disappear, and a film made of high density PTFE is obtained. At this time, the film can be further compressed through a pressure roll. The film made of high density PTFE can also be obtained by pressurizing a PTFE film having a porous structure produced by uniaxially or biaxially extending the unsintered film made of PTFE while heating at a temperature equal to or lower than the melting point. As the PTFE film used for the tube according to the invention, it is preferable to use a film made of PTFE which is extended to form a porous structure and then pressurized to have a high density. The film which is pressurized to have a high density may be sintered before being used. The produced film is generally used after being slit to an appropriate width. The film made of PTFE having a porous structure can be changed from the porous structure to high density PTFE by, for example, spirally winding the film made of PTFE having a porous structure around the outer periphery of the core wire, and then causing the film to pass through a ring-shaped die to pressurize the film.

The PTFE film used for the tube according to the invention may contain a filler or other resins as necessary. Examples of the filler include a filler made of carbon, a metal oxide such as alumina, and a resin, and examples of the other resins include a thermoplastic fluororesin. The filler or the other resins may be used alone or in combination.

The PTFE film used for the tube according to the invention may be implemented by laminating the film made of PTFE and a film made of a thermoplastic fluororesin as described above. A fluororesin used as a material for the film made of a thermoplastic fluororesin is preferably a resin having a melting point lower than a crystal melting point of PTFE, such as tetrafluoroethylene-hexafluoropropylene copolymer (FEP) and tetrafluoroethyleneperfluoroalkyl vinyl ether copolymer (PFA). When the PTFE film is formed by laminating the film made of PTFE and the film made of a thermoplastic fluororesin, a ratio between (sums of) thicknesses of the respective films made of the resin is preferably in a range of (PTFE resin/thermoplastic fluororesin) = 10/1 to 1/1.

The PTFE film used for the tube according to the invention preferably has a thickness of 2 $\mu$m or more and 25 $\mu$m or less, more preferably 3 $\mu$m or more and 25 $\mu$m or less, and still more preferably 3 $\mu$m or more and 20 $\mu$m or less. It is advantageous for characteristics of the tube such as that as the thickness of the film decreases, a step between windings of the film is small when the film is spirally wound, and an influence on a surface of the tube is reduced, but if the thickness of the film is too small, there is a risk that wrinkles or breakages of the film are likely to occur when the film is wound.

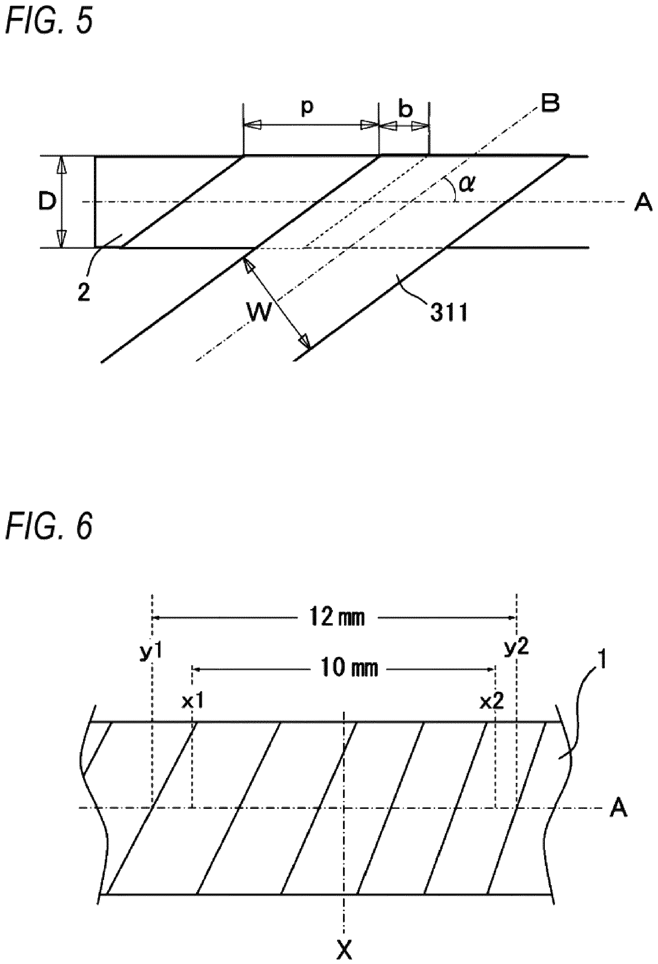

The width of the PTFE film used for the tube according to the invention can be determined according to an inner diameter of the layer formed by spirally winding the PTFE film, the thickness of the layer, the number of windings of the PTFE film, and the like. Fig. 5 is a diagram illustrating parameters to be taken into consideration when determining the width of the PTFE film used for the tube according to the invention. When an outer diameter of the core wire 2 around which the PTFE film is wound (the inner diameter of the layer formed by spirally winding the PTFE film) is denoted by D, a winding angle of a PTFE film 311 is denoted by $\alpha$, a traveling distance when the PTFE film 311 is wound for one winding around the outer periphery of the core wire 2 is denoted by p, and an overlapping amount of the wound PTFE film 311 is denoted by b, a width W of the film 311 is obtained by the following formula. Here, the winding angle $\alpha$ of the film is an angle sandwiched between a center axis A of the core wire 2 and a center line B along a longitudinal direction of the PTFE film 311, and is an angle larger than 0 degrees and smaller than 90 degrees.

$$p = \pi D / \tan\alpha$$

$$W = (p + b)\sin\alpha$$

The tube according to the invention preferably has an endothermic peak in a range of 380°C $\pm$ 10°C in a temperature increasing process of differential scanning calorimetry (DSC). When DSC is performed on a molded article of PTFE, two endothermic peaks on a low temperature side and a high temperature side may be observed due to a difference in a crystal structure. In general, the endothermic peak on the high temperature side appearing at around 380°C is considered to be an endothermic peak derived from an extended chain crystal of PTFE. The PTFE film used for the tube according to the invention preferably includes the film made of high density PTFE which is obtained by pressurizing the film having a porous structure obtained by uniaxial or biaxial extension as described above, and the endothermic peak in the range of 380°C $\pm$ 10°C is also observed by extending PTFE.

In the layer formed by spirally winding the PTFE film of the tube according to the invention, the number of windings per length of 10 mm in the longitudinal direction of the tube is preferably 0.3 to 10 windings/10 mm, and more preferably 0.5 to 8 windings/10 mm. In all layers formed by spirally winding the film, the number of windings of the film does not need to be the same, and each layer may be implemented by a different number of windings.

In at least one of the layer formed by spirally winding the PTFE film, a maximum number of windings per length of 10 mm in the longitudinal direction of the tube is different from a minimum number of windings preferably by at least 0.1 windings/10 mm or more, more preferably by 0.5 windings/10 mm or more, and still more preferably by 1.0 winding/10 mm or more. At this time, a change rate of the number of windings per 10 mm of the film in the longitudinal direction of the tube ((((a maximum value of the number of windings per 10 mm of the film) - (a minimum value of the number of windings per 10 mm of the film))/(the minimum value of the number of windings per 10 mm of the film)) $\times$ 100) (%) is preferably in a range of 10% to 800%, and more preferably in a range of 50% to 700%. The difference between the maximum number of windings of the film and the minimum number of windings or the change rate of the number of windings may be different in each layer, or a range or a position at which the number of windings in the longitudinal direction of the tube is changed may be different in each layer. By changing the number of windings of the film in the longitudinal direction of the tube, the flexibility can be adjusted in the longitudinal direction of the tube. In the tube according to the invention, the tensile strength is maintained in the entire tube. For example, in each embodiment illustrated in Fig. 2, the right side of the tube is high in tensile strength, and the left side of the tube is improved in flexibility while the tensile strength is maintained.

A method for producing the tube according to the invention will be described in more detail in the following examples. The following examples are merely examples of the invention, and are not intended to limit the content of the invention.

Examples

Example 1

<Production of Tube>

A core wire having an outer diameter of 1.0 mm was prepared. It was confirmed that an outer diameter of a left end portion of the core wire was 0.5 mm, and the outer diameter changed at an angle of 0.72 degrees from a portion having an outer diameter of 1.0 mm toward the left end portion. As a PTFE film of a first layer, a film made of PTFE having a thickness of 7 μm was prepared. As a PTFE film of a second layer, a film obtained by laminating a film made of PTFE having a thickness of 6 μm and a film made of PFA having a thickness of 8 μm was prepared.

The prepared core wire was fed out at a constant speed, and the PTFE film of the first layer was spirally wound on the outer periphery of the portion having an outer diameter of 1.0 mm of the core wire in a manner that the number of windings per length of 10 mm in a longitudinal direction was 0.85 windings/10 mm. In a portion in which the outer diameter of the core wire was changed from the portion having an outer diameter of 1.0 mm to the left end portion, the PTFE film was wound in a manner that the number of windings per length of 10 mm in the longitudinal direction of the left end portion was 3.50 windings/10 mm by changing a winding speed of the PTFE film.

The second layer was laminated on the core wire on which the first layer was formed. Specifically, in the portion having an outer diameter of 1.0 mm of the core wire, the PTFE film of the second layer was spirally wound in a manner that the number of windings per length of 10 mm in the longitudinal direction was 0.80 windings/10 mm, and in the portion in which the outer diameter of the core wire changed from the portion having an outer diameter of 1.0 mm to the left end portion, the PTFE film of the second layer was spirally wound in a manner that the number of windings per length of 10 mm in the longitudinal direction of the left end portion was 3.25 windings/10 mm by changing a winding speed of the PTFE film.

The core wire on which the PTFE films of the first layer and the second layer were laminated was caused to pass through an oven heated to 380°C, sintered, and air-cooled. Thereafter, a tube was obtained by extending only the core wire to reduce the outer diameter, and removing the core wire from the laminated and sintered PTFE films.

A maximum number of windings per length of 10 mm in the longitudinal direction of the produced tube was different from a minimum number of windings by 2.65/10 mm in the first layer and 2.45 windings/10 mm in the second layer.

<Differential Scanning Calorimetry (DSC)>

The produced tube sample was subjected to DSC by using DSC3200SA manufactured by NETZSCH JAPAN. 5 mg of the tube was cut out and sealed in a sample pan made of aluminum with a cover, and measurement was performed by increasing a temperature from a room temperature to 400°C at a temperature increasing rate of 10°C/min.

Based on a DSC curve of the tube produced in Example 1, it was confirmed that an endothermic peak appeared at 378.3°C.

<Number of Windings of PTFE Film>

Fig. 6 is a diagram illustrating a method for counting the number of windings of the PTFE film of the tube according to the invention. Fig. 6 is a schematic view of the layer formed by spirally winding the PTFE film when viewed from the side. The "number of windings per length of 10 mm in the longitudinal direction" indicates how many windings the PTFE film is wound in a section having a length of 10 mm on the center axis A of the tube. In Fig. 6, when counting the number of windings at a position X, the number of windings in a range of about 10 mm around X on the center axis A of the tube is counted. Since counting using a lap of a tape is easy, for example, the number of windings between nearest laps y1 and y2 exceeding the range of 10 mm (between x1 and x2) is counted and converted into the number of windings per 10 mm. The number of windings between y1 and y2 is five, and an interval between y1 and y2 is 12 mm. At this time, the number of windings per length of 10 mm in the longitudinal direction is

$$(5 \text{ windings}/12 \text{ mm}) \times 10 \text{ mm} = 4.17 \text{ windings}/10 \text{ mm}.$$

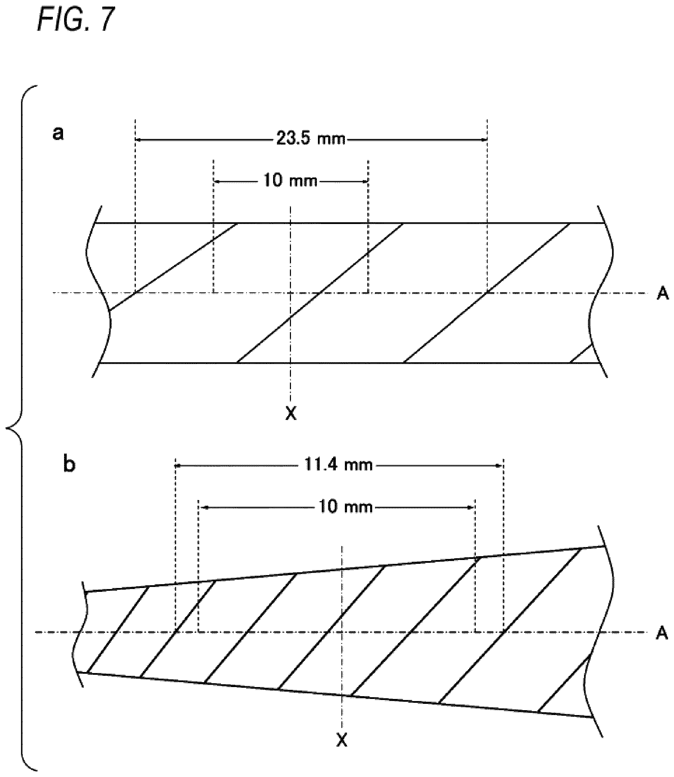

a of Fig. 7 illustrates the first layer in the portion having an outer diameter of 1.0 mm of the core wire of the tube according to Example 1. When calculating as described above, the number of windings per length of 10 mm in the longitudinal direction of the PTFE film is 0.85 windings/10 mm. b of Fig. 7 illustrates the first layer at the left end portion of the tube according to Example 1. When calculating as described above, the number of windings per length of 10 mm in the longitudinal direction of the PTFE film is 3.50 windings/10 mm. In the tube produced in Example 1, the number of windings of the PTFE film is changed between the portion having an outer diameter of 1.0 mm of the core wire and the left end portion, and flexibility of the left end portion of the tube was improved.

<Tensile Test>

A tensile test was performed according to JIS K7127-1999 in an environment of 23°C ± 2°C by using Autograph AGS-1kNX model manufactured by Shimadzu Corporation. The tube was used for a sample of the tensile test as it was, a test speed was 50 mm/min, a distance between chucks was 20 mm, and the distance between chucks was measured as a distance between sample gauge lines (that is, the distance between gauge lines was 20 mm).

Regarding the tube produced in Example 1, a range of 35 mm from the left end portion of the tube was used as a sample of the tensile test of the left end portion of the tube, and a range of 35 mm from a right end portion of the tube was used as a sample of the tensile test of the right end portion of the tube. Both samples were subjected to the tensile test after the number of windings per length of 10 mm in the longitudinal direction in the vicinity of a center between the sample gauge lines was confirmed by the method described above. An outer diameter and a thickness of the tube in the vicinity of the center between the sample gauge lines were measured to obtain a cross-sectional area of the tube, which was defined as a cross-sectional area of the sample of the tensile test. The sample of the tensile test is preferably selected in a manner that a portion having a maximum number of windings and a portion having a minimum number of windings are included in a possible range in the entire tube.

The tensile test was performed by sandwiching and fixing both ends of the sample of the tensile test up to the gauge lines by chucks of a tensile tester.

It was confirmed that tensile strength of the right end portion of the tube according to Example 1 was 247.6 $N/mm^2$, tensile strength of the left end portion in which the number of windings per length of 10 mm in the longitudinal direction was changed was 331.1 $N/mm^2$, and tensile strength of the entire tube was maintained at 100 $N/mm^2$ or more.

Based on the obtained measurement values, a change amount of a tensile stress when a strain of the tube was displaced from 2.5% to 5.0% was confirmed. A stress $\sigma_{2.5}$ at the left end portion of the tube when the strain was 2.5% was 42.8 $N/mm^2$, a stress $\sigma_{5.0}$ when the strain was 5.0% was 69.8 $N/mm^2$, and a change rate E of the tensile stress was E = change amount of stress/displacement amount = $(\sigma_{5.0} - \sigma_{2.5}/0.025) = 27.0/0.025 = 1080$ $N/mm^2$. A stress $\sigma_{2.5}$ of the sample of the tensile test at the right end portion of the tube according to Example 1 when the strain was 2.5% was 39.7 $N/mm^2$, a stress $\sigma_{5.0}$ when the strain was 5.0% was 89.4 $N/mm^2$, and a change rate E of the tensile stress was E = change amount of stress/displacement amount = $(\sigma_{5.0} - \sigma_{2.5}/0.025) = 49.7/0.025 = 1988$ $N/mm^2$. The change rate of the tensile stress is an index when tensile modulus of elasticity of the tube is relatively evaluated. It was confirmed that in the tube according to Example 1, the tensile modulus of elasticity was changed by about twice in the longitudinal direction of the tube by changing the number of windings per length of 10 mm in the longitudinal direction.

Example 2

<Production of Tube>

A core wire having an outer diameter of 1.0 mm was prepared. It was confirmed that an outer diameter of a left end portion of the core wire was 0.75 mm, and the outer diameter changed at an angle of 0.36 degrees from a portion having an outer diameter of 1.0 mm toward the left end portion. As a PTFE film of a first layer, a PTFE resin film having a thickness of 7 μm was prepared. As a PTFE film of a second layer, a PTFE resin film having a thickness of 6 μm was prepared.

The prepared core wire was fed out at a constant speed, and the PTFE film of the first layer was spirally wound on the outer periphery of the portion having an outer diameter of 1.0 mm of the core wire in a manner that the number of windings per length of 10 mm in a longitudinal direction was 0.84 windings/10 mm. In a portion in which the outer diameter of the core wire was changed from the portion having an outer diameter of 1.0 mm to the left end portion, the PTFE film was wound in a manner that the number of windings per length of 10 mm in the longitudinal direction of the left end portion was 1.95 windings/10 mm by changing a winding speed of the PTFE film.

The second layer was laminated on the core wire on which the first layer was formed. Specifically, in a portion having an outer diameter of 1.0 mm of the core wire, the PTFE film of the second layer was spirally wound and laminated in a manner that the number of windings per length of 10 mm in the longitudinal direction was 0.85 windings/10 mm, and in a portion in which the outer diameter of the core wire changed from the portion having an outer diameter of 1.0 mm to the left end portion, the PTFE film of the second layer was spirally wound in a manner that the number of windings per length of 10 mm in the longitudinal direction of the left end portion was 1.88 windings/10 mm by changing a winding speed of the PTFE film.

The core wire on which the PTFE films of the first layer and the second layer were laminated was caused to pass through an oven heated to 380°C, sintered, and air-cooled. Thereafter, a tube was obtained by extending only the core wire to reduce the outer diameter, and removing the core wire from the laminated and sintered PTFE films.

A maximum number of windings per length of 10 mm in the longitudinal direction of the produced tube was different from a minimum number of windings by 1.11/10 mm in the first layer and 1.03 windings/10 mm in the second layer.

The produced tube was subjected to DSC as in Example 1. Based on a DSC curve, it was confirmed that an endothermic peak appeared at 379.7°C.

The produced tube was subjected to a tensile test as in Example 1.

It was confirmed that tensile strength of a right end portion of the tube according to Example 2 was 248.4 N/mm$^2$, tensile strength of the left end portion in which the number of windings per length of 10 mm in the longitudinal direction was changed was 343.2 N/mm$^2$, and tensile strength of the entire tube was maintained. A stress $\sigma_{2.5}$ at the left end portion of the tube when a strain was 2.5% was 44.8 N/mm$^2$, a stress $\sigma_{5.0}$ when the strain was 5.0% was 86.9 N/mm$^2$, and a change rate E of a tensile stress was E = change amount of stress/displacement amount = ($\sigma_{5.0}$ - $\sigma_{2.5}$/0.025) = 42.1/0.025 = 1684 N/mm$^2$. A stress $\sigma_{2.5}$ of the sample of the tensile test at the right end portion of the tube according to Example 1 when the strain was 2.5% was 50.5 N/mm$^2$, a stress $\sigma_{5.0}$ when the strain was 5.0% was 100.4 N/mm$^2$, and a change rate E of the tensile stress was E = change amount of stress/displacement amount = ($\sigma_{5.0}$ - $\sigma_{2.5}$/0.025) = 49.9/0.025 = 1996 N/mm$^2$. It was confirmed that tensile modulus of elasticity of the tube was changed in the longitudinal direction of the tube according to Example 2.

INDUSTRIAL APPLICABILITY

The tube according to the invention is thin and is capable of adjusting flexibility in the longitudinal direction of the tube while maintaining high tensile strength in the entire tube, and is suitable as a liner tube of a tubular body requiring flexibility and smaller diameter.

REFERENCE SIGNS LIST

1: tube according to the present invention
111a, 121a: film
2: core material

**Claims**

1. A tube comprising:

   at least one layer formed by spirally winding a PTFE film, wherein
   the tube has an endothermic peak in a range of 380°C $\pm$ 10°C in a temperature increasing process of DSC of the tube, and
   in at least one of the at least one layer, number of windings per length of 10 mm in a longitudinal direction is not constant.

2. The tube according to claim 1, wherein
   in the at least one layer, at least one layer is spirally wound clockwise, and at least one layer is spirally wound counterclockwise.

3. The tube according to claim 1 or 2, wherein
   the at least one layer has an average thickness of 3 $\mu$m or more and 75 $\mu$m or less.

4. The tube according to any one of claims 1 to 3, wherein
   tensile strength obtained by a tensile test according to JIS K7127-1999 is 100 N/mm$^2$ or more.

5. A tube comprising:

   at least one layer formed by spirally winding a PTFE film, wherein
   the tube has an endothermic peak in a range of 380°C $\pm$ 10°C in a temperature increasing process of DSC of the tube, and
   in at least one of the at least one layer, a maximum number of windings per length of 10 mm in a longitudinal direction is different from a minimum number of windings per length of 10 mm in the longitudinal direction by at least 0.1 windings/10 mm or more.

6.  A tube comprising:

    at least one layer formed by spirally winding a PTFE film, wherein
    the tube has an endothermic peak in a range of 380°C $\pm$ 10°C in a temperature increasing process of DSC of the tube, and
    in at least one of the at least one layer, a maximum number of windings per length of 10 mm in a longitudinal direction is different from a minimum number of windings per length of 10 mm in the longitudinal direction by at least 10% or more.

FIG. 1

FIG. 2

*FIG. 3*

a

121a
111a

2

b

121b
111b

2

*FIG. 4*

221a — 221

2    210    220

211b
211    211a

## FIG. 5

## FIG. 6

*FIG. 7*

**EP 4 176 919 A1**

| | |
|---|---|
| **INTERNATIONAL SEARCH REPORT** | International application No.<br>**PCT/JP2021/025225** |

**A. CLASSIFICATION OF SUBJECT MATTER**

*A61M 25/00*(2006.01)i; *F16L 11/04*(2006.01)i; *F16L 11/24*(2006.01)i
FI: F16L11/04; A61M25/00 500; F16L11/24

According to International Patent Classification (IPC) or to both national classification and IPC

**B. FIELDS SEARCHED**

Minimum documentation searched (classification system followed by classification symbols)

A61M25/00; F16L11/04; F16L11/24

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 2012/0310326 A1 (CAMPBELL, Carey V.) 06 December 2012 (2012-12-06)<br>in particular, paragraphs [0037]-[0038], fig. 1-5 | 1-6 |
| A | JP 9-501759 A (W. L. GORE & ASSOCIATES, INC.) 18 February 1997 (1997-02-18)<br>abstract | 1-6 |
| A | JP 7-1630 A (JAPAN GORE TEX INC.) 06 January 1995 (1995-01-06)<br>in particular, paragraphs [0011]-[0014], fig. 3 | 1-6 |
| A | JP 2012-136020 A (DAIKIN INDUSTRIES, LTD.) 19 July 2012 (2012-07-19)<br>in particular, paragraphs [0062], [0068], | 1-6 |
| A | JP 2019-130880 A (JUNKOSHA INC.) 08 August 2019 (2019-08-08)<br>abstract | 1-6 |
| A | JP 2007-125694 A (TOGAWA RUBBER CO., LTD.) 24 May 2007 (2007-05-24)<br>abstract | 1-6 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **22 September 2021** | **12 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/025225**

| Patent document cited in search report | | | Publication date (day/month/year) | Patent family member(s) | | | Publication date (day/month/year) |
|---|---|---|---|---|---|---|---|
| US | 2012/0310326 | A1 | 06 December 2012 | WO | 97/002791 | A1 | |
| JP | 9-501759 | A | 18 February 1997 | US | 2008/0021533 | A1 | |
| | | | | US | 2003/0073908 | A1 | |
| | | | | WO | 95/005555 | A1 | |
| | | | | abstract | | | |
| JP | 7-1630 | A | 06 January 1995 | EP | 605243 | A1 | |
| | | | | in particular, specification, column 4, line 43 to column 6, line 23, fig. 3 | | | |
| JP | 2012-136020 | A | 19 July 2012 | WO | 2012/077760 | A1 | |
| JP | 2019-130880 | A | 08 August 2019 | (Family: none) | | | |
| JP | 2007-125694 | A | 24 May 2007 | (Family: none) | | | |

Form PCT/ISA/210 (patent family annex) (January 2015)

**EP 4 176 919 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000316977 A
- JP 2013176583 A
- JP 2004340364 A
- JP 6244490 B